# EUROPEAN PATENT APPLICATION

(11) **EP 2 258 328 A1**
(43) Date of publication of application: **08.12.2010**
(21) Application number: 09725889.1
(22) Date of filing: 25.03.2009
(51) Int. Cl.: A61F 13/15, A61F 5/44, A61F 13/49, A61F 13/53

(54) **DIAPER AND URINE RECEIVING PAD FOR DIAPER**

(30) Priority: 28.03.2008 JP 2008088661
(71) Applicant: Unicharm Corporation, Ehime 799-0111 (JP)
(72) Inventor: AOYAGI, Natsuko, Kanonji-shi Kagawa 769-1602 (JP); KENMOCHI, Yasuhiko, Kanonji-shi Kagawa 769-1602 (JP)
(74) Representative: Murgatroyd, Susan Elizabeth
(86) International application number: PCT/JP2009/055980
(87) International publication number: WO 2009/119679

(57) **Abstract**

An effective technique for enhancing ease of mounting a urine absorbing pad on a diaper body is provided in a diaper to be worn by a diaper wearer. The diaper has a urine absorbing pad (120) to be mounted on the diaper body. The urine absorbing pad (120) includes a liquid-permeable sheet (121) provided on a wearer's side, a liquid-impermeable sheet (122) provided on a diaper body side, an absorber (123) disposed between the liquid-permeable sheet (121) and the liquid-impermeable sheet (122) laid one on another, and an annular belt (124) formed in a rear end edge region of the urine absorbing pad (120). When the urine absorbing pad is mounted on the diaper body, an insertion piece of the diaper body is inserted into an opening of the belt (124), so that the urine absorbing pad is positioned with respect to the diaper body.

## Description

### FIELD OF THE INVENTION

The invention relates to a diaper and more particularly, to a technique for providing a diaper in which a urine absorbing pad is mounted on a diaper body.

### BACKGROUND OF THE INVENTION

Diapers for use with a urine absorbing pad have been disclosed. As a known art, Japanese non-examined laid-open Patent Publication No. 2005-95510 discloses a diaper in which displacement of the urine absorbing pad can be prevented by inserting a belt into a hole or guide formed in the back of the urine absorbing pad and fastening the urine absorbing pad to a diaper body via fastening parts on the both ends of the belt. In such a case of use with the urine absorbing pad, however, relative movement of the belt fastened to the diaper body and the urine absorbing pad is allowed, so that the position of the urine absorbing pad may be displaced with respect to the diaper body. Further, at the time of adjustment of the position of the urine absorbing pad, the fastening parts on the both ends of the belt must be disengaged. Thus, the mounting of the urine absorbing pad on the diaper body in proper position is troublesome. Therefore, in designing a diaper of this type in which a urine absorbing pad is mounted on a diaper body, it is required to enhance ease of mounting the urine absorbing pad.

### SUMMARY OF THE INVENTION

It is accordingly an object of the invention to enhance ease of mounting a urine absorbing pad on a diaper body within a diaper.

The above-described object can be achieved by the claimed invention. The invention can be applied to diapers of the type for use with a urine absorbing pad for a diaper which is formed separately from a diaper body.

A representative diaper according to the invention includes at least a diaper body and a urine absorbing pad to be mounted on the diaper body. The diaper body is provided as a member for covering wearer's skin with the urine absorbing pad therebetween and forms an outer surface of the diaper when the diaper is worn by a wearer. The urine absorbing pad includes a liquid-permeable sheet, a liquid-impermeable sheet, an absorber and an opening. The liquid-permeable sheet is provided on the wearer's side. The liquid-permeable sheet typically comprises nonwoven fabric or porous film made of thermoplastic polymer. The liquid-impermeable sheet is provided on the diaper body side. The liquid-impermeable sheet typically comprises film or nonwoven fabric made of thermoplastic polymer, or a composite sheet formed by the film and the nonwoven fabric laid one on another in layers. The absorber is disposed between the liquid-permeable sheet and the liquid-impermeable sheet laid one on another. The absorber has a liquid absorbing function of absorbing and holding liquid such as urine, and typically comprises polymer materials having a liquid absorbing function.

The urine absorbing pad particularly features the opening formed therein. The opening here may be formed by forming a loop in an elongate belt or string, or by a slit formed in the urine absorbing pad. With such a construction, when the urine absorbing pad is mounted on the diaper body, an insertion piece of the diaper body is inserted into the opening, so that the urine absorbing pad is positioned with respect to the diaper body. Thus, the urine absorbing pad can be easily positioned with respect to the diaper body, so that ease of mounting the urine absorbing pad on the diaper body can be enhanced.

According to a further aspect of the invention, preferably, the opening formed in the urine absorbing pad is defined by an annular member provided in an edge region of the urine absorbing pad. The annular member is typically formed by a belt- or string-type member. With such a construction, a diaper is provided in which the annular member provided in an edge region of the urine absorbing pad is used to position the urine absorbing pad with respect to the diaper body.

According to a further aspect of the invention, preferably, an insertion piece fastener part is disposed between the annular member and the insertion piece inserted into the opening to removably fasten the insertion piece to the annular member. As the insertion piece fastener part, typically, hook and loop fasteners or hook tapes can be used. With such a construction, the insertion piece of the diaper body is inserted into the opening and can be reliably held on the annular member side by the insertion piece fastener part.

According to a further aspect of the invention, preferably, the annular member is provided in a rear end region of the edge region of the urine absorbing pad which faces a wearer's back and has an extending piece extending from the annular member. With such a construction, in the process of putting on the diaper, the urine absorbing pad disposed on the inside surface of the diaper body can be easily adjusted in position by pulling it up from the outside of the diaper body via the extending piece of the annular member.

According to a further aspect of the invention, preferably, the annular member comprises an elongate belt and has belt fastener parts to removably fasten both ends of the belt to the edge region of the urine absorbing pad. With such a construction, a belt can be retrofitted to a urine absorbing pad of the type having no such belt, via the belt fastener parts.

According to a further aspect of invention, preferably, a urine absorbing pad fastener part is disposed between the diaper body and the urine absorbing pad to removably fasten the urine absorbing pad to the diaper body. With such a construction, displacement of the urine absorbing pad with respect to the diaper body can be prevented by the urine absorbing pad fastener part.

According to a further aspect of invention, preferably, the opening is provided in a rear end region of the urine absorbing pad which faces a wearer's back and the urine absorbing pad fastener part is provided in a front end region of the urine absorbing pad which faces a wearer's front. With such a construction, the function of holding the urine absorbing pad on the diaper body can be realized with a simpler structure by inserting the insertion piece of the diaper body into the opening in the rear end region of the urine absorbing pad and by using the urine absorbing pad fastener part in the front end region of the urine absorbing pad.

A representative urine absorbing pad for a diaper according to the invention is mountable on a diaper body and has the same construction as the urine absorbing pad of the above-described diaper. Therefore, by use of such a urine absorbing pad for a diaper, the urine absorbing pad can be easily positioned with respect to the diaper body, so that ease of mounting the urine absorbing pad on the diaper body can be enhanced.

According to a further aspect of the invention, preferably, the opening is defined by an annular member provided in the edge region of the urine absorbing pad. With such a construction, the annular member provided in the edge region can be used to position the urine absorbing pad with respect to the diaper body.

According to a further aspect of the invention, preferably, an insertion piece fastener part is disposed between the annular member and the insertion piece inserted into the opening to removably fasten the insertion piece to the annular member. With such a construction, the insertion piece of the diaper body is inserted into the opening and can be reliably held on the annular member side by the insertion piece fastener part.

According to a further aspect of the invention, preferably, the annular member is provided in a rear end region of the edge region of the urine absorbing pad which faces a wearer's back and has an extending piece extending from the annular member. With such a construction, in the process of putting on the diaper, the urine absorbing pad disposed on the inside surface of the diaper body can be easily adjusted in position by pulling it up from the outside of the diaper body via the extending piece of the annular member.

According to a further aspect of the invention, preferably, the annular member comprises an elongate belt and has belt fastener parts to removably fasten both ends of the belt to the edge region of the urine absorbing pad. With such a construction, a belt can be retrofitted to a urine absorbing pad of the type having no such belt, via the belt fastener parts.

According to a further aspect of the invention, preferably, a urine absorbing pad fastener part is disposed between the diaper body and the urine absorbing pad to removably fasten the urine absorbing pad to the diaper body. With such a construction, displacement of the urine absorbing pad with respect to the diaper body can be prevented by the urine absorbing pad fastener part.

According to a further aspect of the invention, preferably, the opening is provided in a rear end region of the urine absorbing pad which faces a wearer's back and the urine absorbing pad fastener part is provided in a front end region of the urine absorbing pad which faces a wearer's front. With such a construction, the function of holding the urine absorbing pad on the diaper body can be realized with a simpler structure by inserting the insertion piece of the diaper body into the opening in the rear end region of the urine absorbing pad and by using the urine absorbing pad fastener part in the front end region of the urine absorbing pad.

As described above, according to the invention, in a diaper to be worn by a diaper wearer, particularly, an opening is provided in a urine absorbing pad, and when the urine absorbing pad is mounted on the diaper body, an insertion piece of the diaper body is inserted into the opening, so that the urine absorbing pad is positioned with respect to the diaper body. With this construction, the urine absorbing pad can be easily positioned with respect to the diaper body, so that ease of mounting the urine absorbing pad on the diaper body can be enhanced.

### BRIEF DESCRIPTION OF THE DRAWINGS

FIG. 1 is a perspective view showing a diaper 100 according to an embodiment of this invention, as viewed from a wearer's skin contact side.
FIG. 2 is a sectional view showing a sectional structure of the diaper 100 and taken along line A-A in FIG. 1.
FIG. 3 is a perspective view showing the diaper body 110 shown in FIG. 1.
FIG. 4 is a perspective view showing a urine absorbing pad 120 shown in FIG. 1.
FIG. 5 is a plan view of the urine absorbing pad 120 as viewed from the back.
FIG. 6 is a plan view showing a urine absorbing pad 220 according to another embodiment as viewed from the back.
FIG. 7 is a plan view showing a urine absorbing pad 320 according to another embodiment as viewed from the back.
FIG. 8 is a side view showing the diaper 100 according to the present embodiment in the state worn by a wearer, and showing the state in which belts 124 are not yet pulled up.
FIG. 9 is a side view showing the diaper 100 according to the present embodiment in the state worn by a wearer, and showing the state in which the belts 124 are pulled up.

### DEATAILED DESCRIPTION OF THE INVENTION

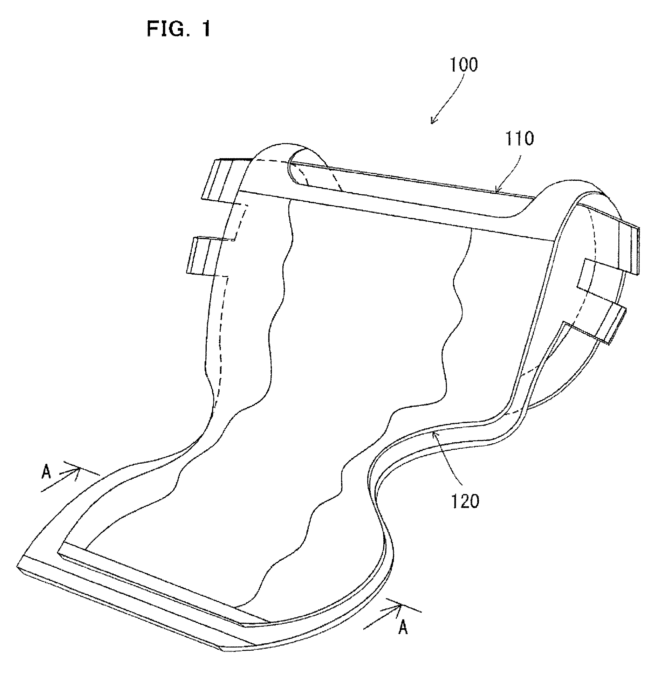

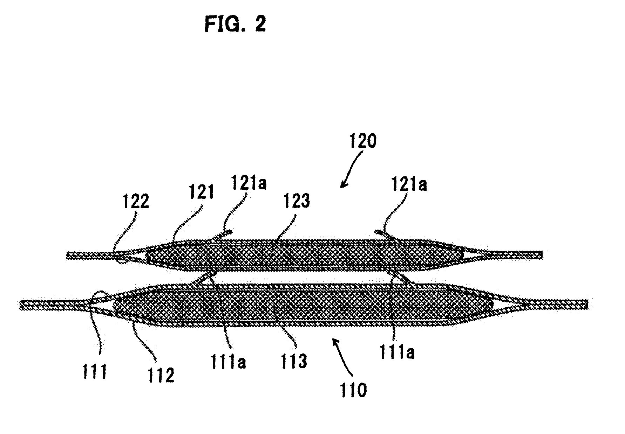

The structure of a diaper 100 according to a representative embodiment of the invention is now described with reference to the drawings. The diaper 100 is designed as a diaper to be worn by a diaper wearer. FIG. 1 is a perspective view showing the diaper 100 according to this embodiment, as viewed from a wearer's skin contact side. FIG. 2 is a sectional view showing a sectional structure of the diaper 100 and taken along line A-A in FIG. 1.

As shown in FIG. 1, the diaper 100 includes a diaper body 110 and a urine absorbing pad 120. The urine absorbing pad 120 is slightly smaller than the diaper body 110 and laid on the diaper body 110 on the wearer's side of the diaper body 110. The diaper 100 is in the form of a flat sheet at the time of purchase or in the unused state, and in use, the diaper takes a three-dimensional form when worn by a wearer. In this embodiment, one side of the diaper which comes in contact with wearer's skin is taken as the "front side" or the "front", while the opposite side which forms an outer surface of the diaper when it is worn by a wearer is taken as the "back side" or "back". Further, the side of the diaper which is set on a front of the wearer is taken as the "front end side", while the side of the diaper which is set on a back of the wearer is taken as the "rear end side".

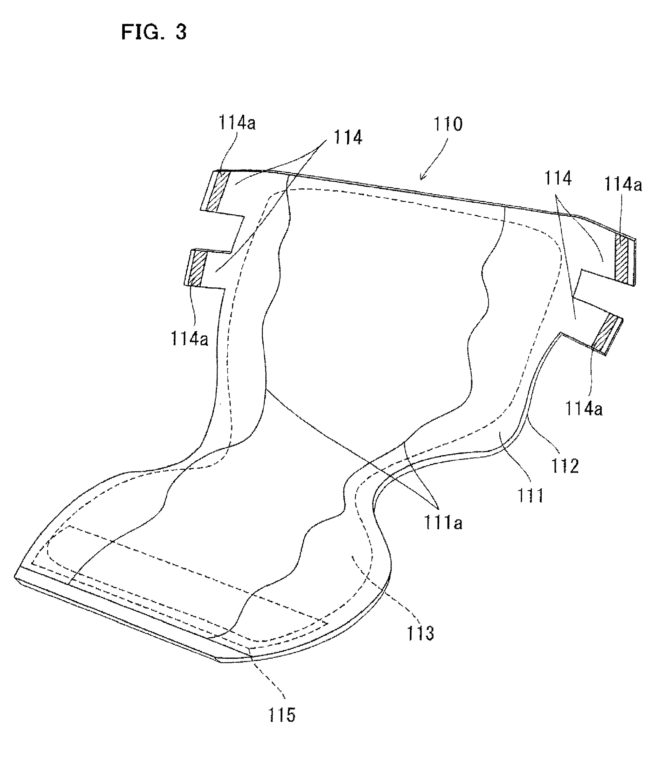

Referring to FIGS. 2 and 3, the structure of the diaper body 110 shown in FIG. 1 is described. FIG. 3 is a perspective view showing the diaper body 110 shown in FIG. 1. As shown in FIGS. 2 and 3, in this embodiment, the diaper body 110 has a front sheet 111, a back sheet 112 and an absorber 113 disposed between the front sheet 111 and the back sheet 112. The diaper body 110 here is a feature that corresponds to the "diaper body" according to this invention.

Each of the front sheet 111 and the back sheet 112 is formed as a liquid-permeable sheet and typically comprises nonwoven fabric or porous film made of thermoplastic polymer. Further, a pair of right and left three-dimensional gatherings 111a are provided on the front sheet 111 and raised inward from the both sides of the front sheet 111. The gatherings 111a have a function of preventing a solid matter or the like from running off laterally outward. The absorber 113 has a liquid absorbing function of absorbing and holding liquid such as urine, and typically comprises polymer materials having a liquid absorbing function.

Further, a plurality of extending pieces 114 are formed on the both sides of a rear end portion of the diaper body 110 and extend outward. Each of the extending pieces 114 has a fastener part 114a which forms a fastening structure in cooperation with a fastening part 115 provided on a front end portion of the back sheet 112 of the diaper body 110. As the fastener part 114a, hook and loop fasteners or hook tapes, or adhesive tapes may be appropriately used. With such a construction, the extending pieces 114 are fastened to the fastening part 115 via the fastener parts 114a, so that the diaper body 110 takes a pants-shaped three-dimensional form and can retain this three-dimensional form. The extending piece 114 here is a feature that corresponds to the "insertion piece of the diaper body" according to this invention.

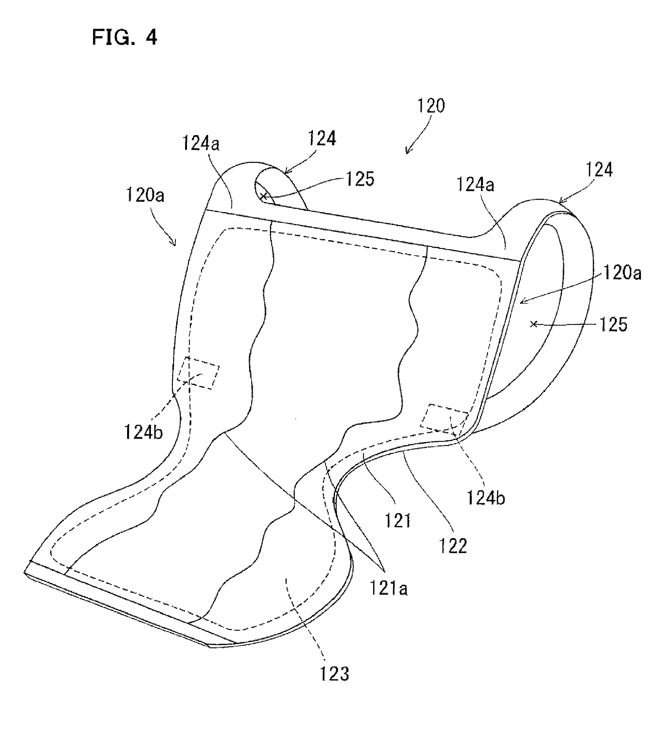

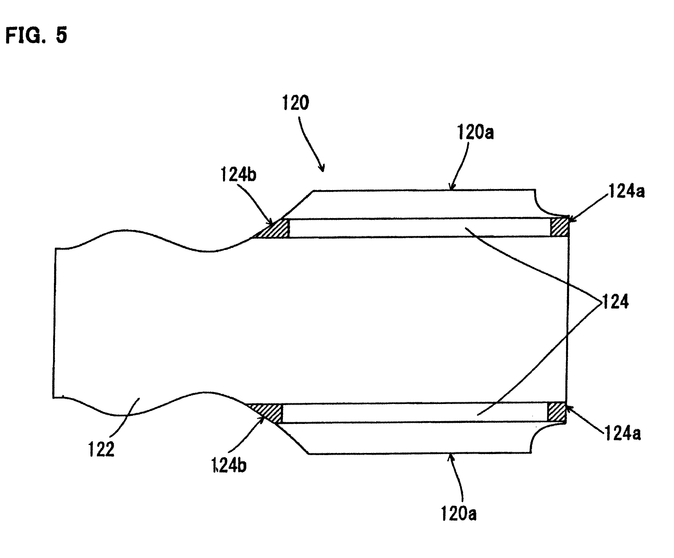

Referring to FIGS. 2, 4 and 5, the structure of the urine absorbing pad 120 shown in FIG. 1 is described. FIG. 4 is a perspective view of the urine absorbing pad 120 shown in FIG. 1, and FIG. 5 is a plan view of the urine absorbing pad 120 as viewed from the back. As shown in FIGS. 2, 4 and 5, the urine absorbing pad 120 of this embodiment has a front sheet 121, a back sheet 122 and an absorber 123 disposed between the front sheet 121 and the back sheet 122.

The front sheet 121 is formed as a liquid-permeable sheet and has a function of allowing liquid such as urine to pass down to the absorber 123. The front sheet 121 typically comprises nonwoven fabric or porous film made of thermoplastic polymer. The front sheet 121 here is a feature that corresponds to the "liquid-permeable sheet" according to this invention. Further, a pair of right and left three-dimensional gatherings 121a similar to the above-described three-dimensional gatherings 111a are provided on the front sheet 121 and raised inward from the both sides of the front sheet 111. The gatherings 121a have a function of preventing a solid matter or the like from running off laterally outward. The back sheet 122 is formed as a liquid-impermeable sheet and has a function of preventing liquid such as urine to pass down to the back side. The back sheet 122 typically comprises film or nonwoven fabric made of thermoplastic polymer, or a composite sheet formed by the film and the nonwoven fabric laid one on another in layers. The back sheet 122 here is a feature that corresponds to the "liquid-impermeable sheet" according to this invention. Like the above-described absorber 113, the absorber 123 has a liquid absorbing function of absorbing and holding liquid such as urine, and typically comprises polymer materials having a liquid absorbing function. The absorber 123 here is a feature that corresponds to the "absorber" according to this invention.

As shown in FIGS. 4 and 5, a pair of elongate belts 124 are provided on right and left edges 120a of a rear end region of the urine absorbing pad 120. Both ends 124a, 124b of each of the belts 124 are fastened to the back of the urine absorbing pad 120, so that the belt 124 is formed into a loop and thus defines an opening 125. In this embodiment, one end 124a of the belt 124 is fixed to the rear end of the urine absorbing pad 120, and the other end 124b of the belt 124 is fixed to the urine absorbing pad 120 nearly at its middle in the longitudinal direction. The belt 124 can be formed of the same material as the front sheet 121, and preferably, the both belt ends 124a, 124b may be fixed by using adhesives or by fusion bonding. With such a construction, the extending pieces 114 of the diaper body 110 can be inserted into the openings 125 of the belts 124, and in the inserted state, the diaper body 110 and the urine absorbing pad 120 can be easily and reliably positioned with respect to each other. Further, it may be constructed such that the both belt ends 124a, 124b of the belts 124 are fixed to the front sheet 121 of the urine absorbing pad 120. The "belt" and the "opening" here are features that correspond to the "annular member" and the "opening", respectively, according to this invention.

Each of the belts 124 may be preferably formed of stretchy nonwoven fabric. In this case, the nonwoven fabric itself may be stretchy, or the stretchiness may be provided by using the nonwoven fabric in combination with an elastic material. By use of the stretchy belts 124, insertion of the extending pieces 114 into the openings 125 of the belts 124 is made easier, and the belts 124 can be effectively prevented from becoming displaced, slacked or wrinkled.

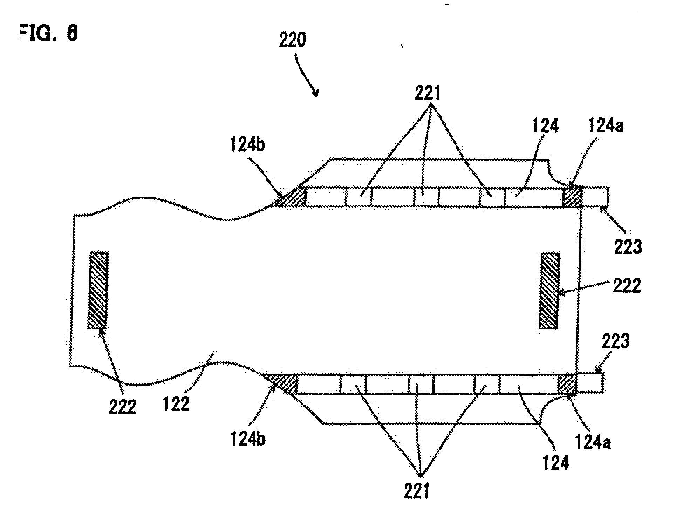

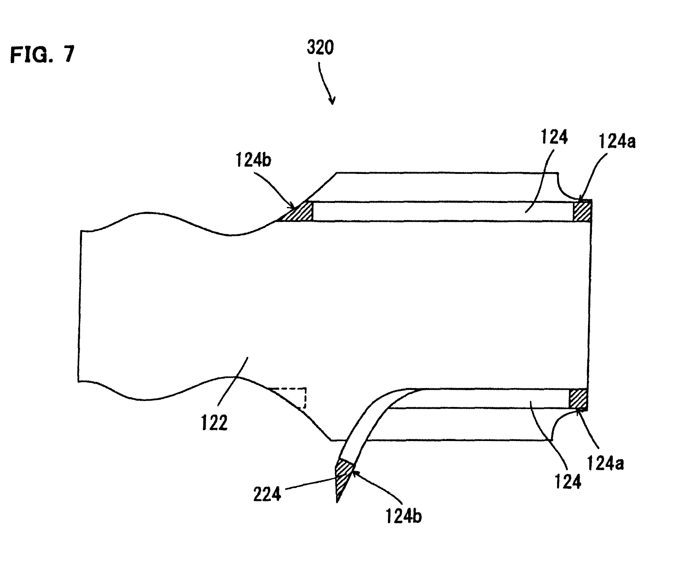

Preferably, the urine absorbing pad 120 according to this embodiment may further have first to fourth features which will be described below in detail. It is preferable for the urine absorbing pad 120 to have all of the first to fourth features, but it can have at least one of the four features as necessary. The first to fourth features are now explained with reference to FIGS. 6 and 7. FIG. 6 is a plan view showing a urine absorbing pad 220 according to another embodiment as viewed from the back, and FIG. 7 is a plan view showing a urine absorbing pad 320 according to another embodiment as viewed from the back. Components or elements in FIGS. 6 and 7 which are substantially identical to those shown in FIG. 5 are given like numerals and will not be described.

### (First Feature)

As for the first feature, the construction of the urine absorbing pad 220 shown in FIG. 6 is referred to. In the urine absorbing pad 220, each of the belts 124 has a first fastener part 221 for fastening and holding the extending pieces 114 of the diaper body 110 which are inserted into an opening (similar to the above-described opening 125) defined by the belt 124. The first fastener part 221 here is a feature that corresponds to the "insertion piece fastener part" according to this invention. With such a construction, the extending pieces 114 of the diaper body 110 which are inserted into the opening 125 of the belt 124 can be reliably fastened and held on the belt 124 side by the first fastener part 221. One or more first fastener parts 221 may be provided on each of the belts 124 as necessary. Particularly, in order to make it possible to adjust the holding position of the extending pieces 114, hook and loop fasteners or hook tapes which can removably hold the extending pieces 114 are preferably used as the first fastener part 221.

### (Second Feature)

As for the second feature, the construction of the urine absorbing pad 220 shown in FIG. 6 is referred to. The urine absorbing pad 220 has a second fastener part 222 on each of front and rear end portions of the urine absorbing pad 220. The second fastener part 222 may be provided on at least one of the front and rear end portions of the urine absorbing pad 220 as necessary. The second fastener part 222 forms a fastening structure in cooperation with the diaper body 110. The second fastener part 222 here is a feature that corresponds to the "urine absorbing pad fastener part" according to this invention. With such a construction, displacement of the urine absorbing pad 220 with respect to the diaper body 110 can be prevented by the second fastener part 222. As the second fastener part 222, hook and loop fasteners or hook tapes, or adhesive tapes may be appropriately used. Particularly, in order to make it possible to adjust the position of the urine absorbing pad 220 with respect to the diaper body 110, removable hook and loop fasteners or hook tapes are preferably used as the second fastener part 222.

### (Third Feature)

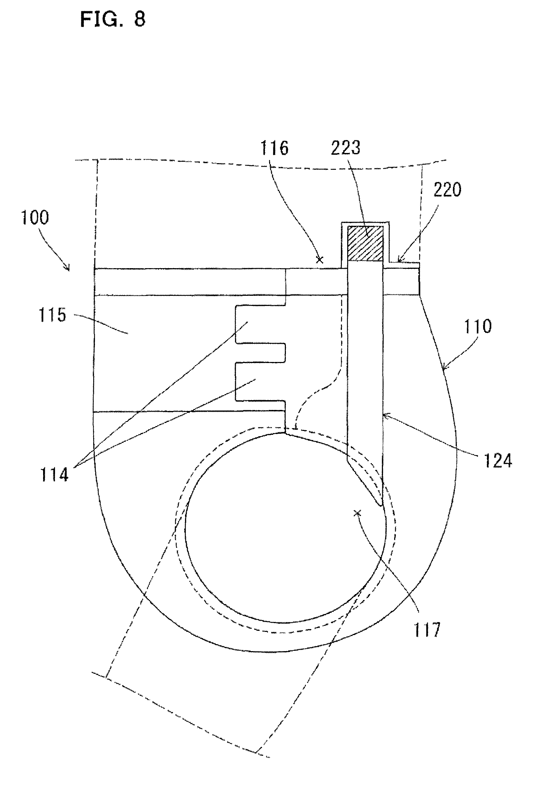

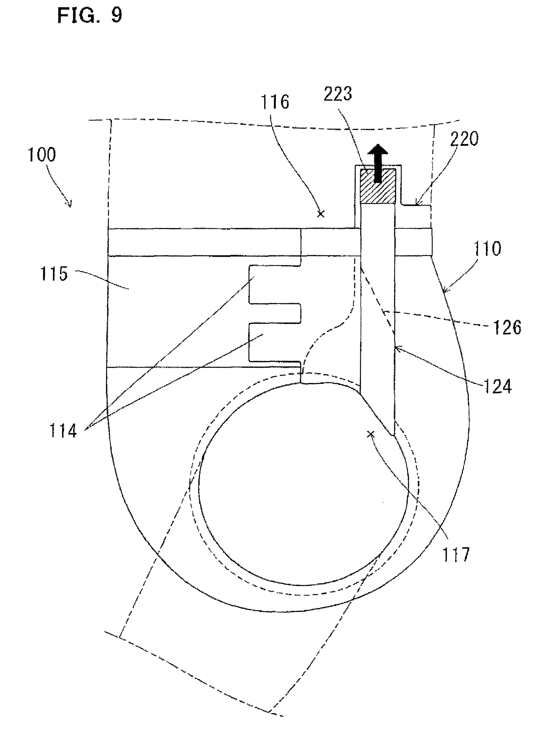

As for the third feature, the construction of the urine absorbing pad 220 shown in FIG. 6 is referred to. The urine absorbing pad 220 has an extending piece 223 extending from the rear end edge of the urine absorbing pad 220 in the direction in which the pad is pulled up (rightward as viewed in FIG. 6). The extending piece 223 here is a feature that corresponds to the "extending piece" according to this invention. With such a construction, a diaper wearer or a helper of the wearer can hold and pull up the extending piece 223 extending from the edge of the urine absorbing pad 220. Therefore, in the process of putting on the diaper 100, the urine absorbing pad 220 disposed on the inside surface of the diaper body 110 can be easily adjusted in position by pulling it up from the outside of the diaper body 110. This adjustment is described below in further detail with reference to FIGS. 8 and 9.

The extending piece 223 may be provided on at least one of the front sheet 121 and the back sheet 122, or on the belt 124 itself. In the former case in which the extending piece 223 is provided on at least one of the front sheet 121 and the back sheet 122, the extending piece 223 may be formed by protruding part of the front sheet 121 or the back sheet 122, or the extending piece 223 may be formed by connecting a separate member to the front sheet 121 or the back sheet 122. In the case in which the extending piece 223 is provided on the belt 124, the extending piece 223 may be formed by protruding part of the belt 124, or by connecting a separate member to the belt 124. In view of the appearance, the extending piece 223 may be preferably formed in a square shape having each side of about 40 to 60 mm. Further, one or more extending pieces 223 may be provided on the urine absorbing pad.

Further, various kinds of information relating to the diaper 100 can be indicated on the extending piece 223. With such a construction, a diaper wearer or a helper of the wearer can visually check various kinds of information. The information here includes information relating to the absorber 123 (the amount of absorption, the kind and the installation position), indication relating to operation of pulling the extending piece 223 (the pulling direction, the amount of pulling, the position to pull) and indication of the front and the back of the diaper 100.

### (Fourth Feature)

As for the fourth feature, the construction of the urine absorbing pad 320 shown in FIG. 7 is referred to. The urine absorbing pad 320 has third fastener parts 224 on both belt ends 124a, 124b of each of the belts 124. In FIG. 7, for the sake of convenience of explanation, a third fastener part 224 is shown provided only on the belt end 124b of one of the belts 124. The third fastener part 224 forms a fastening structure in cooperation with the urine absorbing pad 320. The third fastener part 224 here is a feature that corresponds to the "belt fastener part" according to this invention. With such a construction, a belt 124 can be retrofitted to a urine absorbing pad of the type having no such belt 124, via the third fastener parts 224. As the fastener part 224, hook and loop fasteners or hook tapes, or adhesive tapes may be appropriately used. Particularly, in order to make it possible to adjust the position of the belt 124 with respect to the urine absorbing pad 320, removable hook and loop fasteners or hook tapes are preferably used as the fastener part 224.

When hook and loop fasteners or hook tapes having a known construction are used as the above-described first to third fastener parts 221, 222, 224, specifically, mushroom heads of tiny mushroom type (hooks of hook type) are arranged on one side of one tape, and loops are arranged on one side of the other tape. When the both sides are pressed against each other, the mushrooms (or hooks) are engaged with the loops, so that the both sides easily stick to each other. Further, in order to peel off the tapes, the tapes are pulled away from each other so that the mushrooms (or hooks) are disengaged from the loops. Thus, the tapes can be easily peeled off away from each other.

In order to use the above-described diaper 100 on a diaper wearer, first, the urine absorbing pad 120 is laid on the diaper body 110. At this time, preferably, the fastening structure having the second fastening parts 222 described in the second feature is used. Next, the diaper 100 is wrapped around the region of the wearer's laps with the urine absorbing pad 120 inside, and the right and left extending pieces 114 of the diaper body 110 are inserted into the openings 125 of the right and left belts 124. Then the fastener parts 114a of the extending pieces 114 are fastened to the fastening part 115. At this time, the diaper body 110 and the urine absorbing pad 120 can be temporarily positioned with respect to each other by insertion of the fastener parts 114a of the diaper body 110 into the openings 125 of the belts 124. Thereafter, the diaper 100 is put on when pulled up to around the wearer's waist in its entirety.

The effect of the above-described second feature is described with reference to FIGS. 8 and 9. FIGS. 8 and 9 are side views showing the diaper 100 according to this embodiment in the state worn by a wearer. Particularly, FIG. 8 shows the state in which the belts 124 are not yet pulled up, and FIG. 9 shows the state in which the belts 124 are pulled up.

As shown in FIG. 8, in the state in which the diaper 100 is worn by a wearer shown by the two-dot chain line in the drawing, the above-described urine absorbing pad 220 is laid on the inside surface of the diaper body 110, and the extending pieces 114 of the diaper body 110 which are inserted into the openings of the belts 124 are fastened to the fastening part 115 via the fastener parts 114a. In such a diaper wearing state, in the process of pulling the whole diaper 100 up to around the wearer's waist, the temporarily positioned diaper body 110 and urine absorbing pad 220 may be displaced from each other. Further, in such a diaper wearing state, the belts 124 extend from the urine absorbing pad 220 via a waist opening 116 and leg openings 117 in the pulling-up direction. Further, the extending pieces 223 extend rearward (upward as viewed in FIG. 8) from the edge of the urine absorbing pad 220.

Therefore, in this embodiment, by pulling up the extending pieces 223, the position of the urine absorbing pad 220 with respect to the diaper body 110 can be adjusted after the diaper is worn. Specifically, as shown in FIG. 9, when the extending pieces 223 are pulled up as shown by arrow in the drawing, the urine absorbing pad 220 is pulled up in its entirety via the belts 124. Further, at this time, the belts 124 are engaged with the edges of the leg openings 117 of the diaper body 110, so that a load is also applied to the diaper body 110 in the pulling-up direction. Therefore, the urine absorbing pad 220 can be pulled up in a well-balanced manner together with the diaper body 110 in such a manner that the urine absorbing pad 220 is prevented from becoming displaced or separated too much from the diaper body 110. Particularly, by provision of the pair of belts 124 on the right and left end portions of the urine absorbing pad 220, the pad can be easily balanced in the lateral direction when it is pulled up. Further, by pulling up the diaper body 110, a load can also be applied to the urine absorbing pad 220 in the pulling-up direction via the belts 124.

Further, as shown in FIG. 9, preferably, a tear-off line or perforation 126 is formed in advance in each of the belts 124. With such a construction, after use of the diaper, the urine absorbing pad can be easily removed by pulling the belts 124 by hand and tearing them off along the perforation 126.

### (Other Embodiments)

The invention is not limited to the embodiment as described above, but rather, may be added to, changed, replaced with alternatives or otherwise modified. For example, the following provisions can be made in application of this embodiment.

In the above embodiment, the openings 125 into which the extending pieces 114 of the diaper body 110 are inserted are described as being defined by the annular belts 124 provided on the urine absorbing pad 120 side. In this invention, however, parts corresponding to the openings 125 may also be realized by a different structure having a slit formed in the urine absorbing pad 120.

Further, in the above embodiment, the pair of belts 124 are described as being provided on the edges 120a of the rear end region of the urine absorbing pad 120. In this invention, however, one or more belts 124 can be provided. Further, in this invention, the belts 124 may also be provided on edges other than the edges 120a of the urine absorbing pad 120. For example, one or more belts 124 can be provided on at least one of the edge of the rear end region and the edge of the front end region of the urine absorbing pad 120.

Further, in the above embodiment, the diaper is described which is in the form of a flat sheet at the time of purchase or in the unused state and takes a three-dimensional form in use when worn by a wearer. However, this invention can also be applied to a diaper which is three-dimensionally formed in advance.

### Description of Numerals

- 100: diaper
- 110: diaper body
- 111: front sheet
- 111a: three-dimensional gathering
- 112: back sheet
- 113: absorber
- 114: extending piece
- 114a: fastener part
- 115: fastening part
- 116: waist opening
- 117: leg opening
- 120, 220, 320: urine absorbing pad
- 120a: edge
- 121: front sheet
- 121a: three-dimensional gathering
- 122: back sheet
- 123: absorber
- 124: belt
- 124a, 124b: belt end
- 125: opening
- 126: perforation
- 221: first fastener part
- 222: second fastener part
- 223: extending piece
- 224: third fastener part

## Claims

1. A diaper comprising a diaper body and a urine absorbing pad mounted on the diaper body,
wherein the urine absorbing pad includes a liquid-permeable sheet provided on a wearer's side, a liquid-impermeable sheet provided on a diaper body side, an absorber disposed between the liquid-permeable sheet and the liquid-impermeable sheet laid one on another, and an opening formed in the urine absorbing pad, wherein, when the urine absorbing pad is mounted on the diaper body, an insertion piece of the diaper body is inserted into the opening, so that the urine absorbing pad is positioned with respect to the diaper body.

2. The diaper as defined in claim 1, wherein the opening is defined by an annular member provided in an edge region of the urine absorbing pad.

3. The diaper as defined in claim 2, wherein an insertion piece fastener part is disposed between the annular member and the insertion piece inserted into the opening so as to removably fasten the insertion piece to the annular member.

4. The diaper as defined in claim 2 or 3, wherein the annular member is provided in a rear end region of the edge region of the urine absorbing pad which faces a wearer's back and has an extending piece extending from the annular member.

5. The diaper as defined in any one of claims 2 to 4, wherein the annular member comprises an elongate belt and has belt fastener parts to removably fasten both ends of the belt to the edge region of the urine absorbing pad.

6. The diaper as defined in any one of claims 1 to 5, wherein a urine absorbing pad fastener part is disposed between the diaper body and the urine absorbing pad to removably fasten the urine absorbing pad to the diaper body.

7. The diaper as defined in claim 6, wherein the opening is provided in a rear end region of the urine absorbing pad which faces a wearer's back and the urine absorbing pad fastener part is provided in a front end region of the urine absorbing pad which faces a wearer's front.

8. A urine absorbing pad for a diaper mountable on a diaper body, the urine absorbing pad comprising:
a liquid-permeable sheet provided on a wearer's side, a liquid-impermeable sheet provided on a diaper body side, an absorber disposed between the liquid-permeable sheet and the liquid-impermeable sheet laid one on another, and an opening formed in an edge region of the urine absorbing pad, wherein, when the urine absorbing pad is mounted on the diaper body, an insertion piece of the diaper body is inserted into the opening, so that the urine absorbing pad is positioned with respect to the diaper body.

9. The urine absorbing pad as defined in claim 8, wherein the opening is defined by an annular member provided in the edge region of the urine absorbing pad.

10. The urine absorbing pad as defined in claim 9, wherein an insertion piece fastener part is disposed between the annular member and the insertion piece inserted into the opening to removably fasten the insertion piece to the annular member.

11. The urine absorbing pad as defined in claim 9 or 10, wherein the annular member is provided in a rear end region of the edge region of the urine absorbing pad which faces a wearer's back and has an extending piece extending from the annular member.

12. The urine absorbing pad as defined in any one of claims 9 to 11, wherein the annular member comprises an elongate belt and has belt fastener parts to removably fasten both ends of the belt to the edge region of the urine absorbing pad.

13. The urine absorbing pad as defined in any one of claims 9 to 12, wherein a urine absorbing pad fastener part is disposed between the diaper body and the urine absorbing pad to removably fasten the urine absorbing pad to the diaper body.

14. The urine absorbing pad as defined in claim 13, wherein the opening is provided in a rear end region of the urine absorbing pad which faces a wearer's back and the urine absorbing pad fastener part is provided on a front end region of the urine absorbing pad which faces a wearer's front.
